# EUROPÄISCHE PATENTSCHRIFT

(11) **EP 0 864 653 B1**
(45) Veröffentlichungstag und Bekanntmachung des Hinweises auf die Patenterteilung: **03.03.2004**
(21) Anmeldenummer: 98103599.1
(22) Anmeldetag: 02.03.1998
(51) Int. Cl.: C12P 9/00, C12P 17/12, C07F 9/38

(54) **Verfahren zur Herstellung von 4-(4-(4-(Hydroxydiphenyl)-1-piperidinyl)-1-hydroxybutyl)-alpha,alpha-dimenthylphenylessigsäure und phosphorylierter Derivate**
Process for production of 4-(4-(4-(hydroxydiphenyl)-1-piperidinyl)-1-hydroxybutyl)-alpha, alpha-dimethylphenyl acetic acid and phosphorylated derivatives
Procédé de production d'acide 4-(4-(4-(hydroxydiphényl)-1-piperidinyl)-1-hydroxybutyl)-alpha,alpha-dimethylphénylacétique et dérivés phosphorylés

(30) Priorität: 11.03.1997 DE 19709898; 21.11.1997 DE 19751498
(43) Veröffentlichungstag der Anmeldung: 16.09.1998
(73) Patentinhaber: Aventis Pharmaceuticals Inc., Bridgewater, New Jersey 08807 (US)
(72) Erfinder: Meiwes, Johannes, Dr., 65510 Idstein (DE); Worm, Manfred, Dr., 65207 Wiesbaden (DE)
(74) Vertreter: Then, Johann, Dr.

(56) Entgegenhaltungen:
- DE-A- 2 303 306
- DE-A- 4 034 218
- US-A- 4 285 957
- US-A- 5 204 249
- PATENT ABSTRACTS OF JAPAN vol. 015, no. 060 (C-0805), 13. Februar 1991 (1991-02-13) & JP 02 288883 A (SANKYO CO LTD), 28. November 1990 (1990-11-28)

## Beschreibung

Die Erfindung betrifft ein mikrobielles Verfahren zur Herstellung phosphorylierter Derivate aus α-(p-tertiär-Butylphenyl)-4-(α-hydroxy-α-phenylbenzyl)-1-piperidin-butanol (Verbindung 2) sowie deren Verwendung als Arzneimittel. Ferner betrifft die Erfindung betrifft ein mikrobielles Verfahren zur Phosphorylierung von 4-(4-(4-Hydroxydiphenyl)-1-piperidinyl)-1-hydroxybutyl)-α,α-dimethylessigsäure (Verbindung 1).

Es ist bekannt, daß im menschlichen Körper Verbindung 2 in Verbindung 1 umgewandelt wird (DE 23 03 306, US 4,254,129, US 4,285,957, DE 30 07 498). Ferner ist bekannt, daß Ebastin zu Carebastin oxidiert wird mit Hilfe von Mikroorganismen beispielsweise der Gattung Cunninghamella (DE 40 34 218; Schwarz et al. Appl. Microbiol. Biotechnol. (1996) 44: Seiten 731-735).

Es wurde nun gefunden, daß Pilze der Gattungen Cunninghamella oder Absidia die Verbindung 2 selektiv in die Verbindungen der Formel III oder II umwandeln. Die selektive Oxidation nur am p-tertiär-Butylphenylrest der Verbindung 2 zur entsprechenden Carboxylgruppe ist überraschend, da die beiden Hydroxylgruppen in der Verbindung 2, die auch oxydiert werden könnten, nicht oxydiert werden.

Die Erfindung betrifft ein Verfahren zur Herstellung der Verbindung der Formel III, 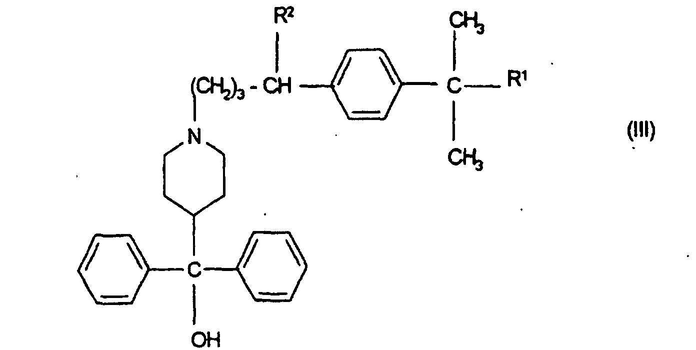 worin
- R¹: für -CH₂-O-P(O)(OH)₂ und R² für -OH steht,
- R¹: für -CH₃ und R² für -O-P(O)(OH)₂ steht oder
- R²: für -O-P(O)(OH)₂ und R¹ für -COOH steht,
dadurch gekennzeichnet, daß α-(p-tertiär-Butylphenyl)-4-(α-hydroxy-α-phenylbenzyl)-1-piperidin-butanol oder gegebenenfalls 4-(4-(4-Hydroxydiphenyl)-1-piperidinyl)-1-hydroxybutyl)-α,α-dimethylessigsäure mit einem Pilz der Gattung Cunninghamella oder Absidia inkubiert wird.

Bevorzugt wird ein Pilz aus der Gruppe Cunninghamella blakesleeana, Cunninghamella elegans und Cunninghamella echinulata, insbesondere ATCC 8688a, DSM 1905, DSM 1908 und ATCC 9244, eingesetzt. Ferner eignen sich Mutanten und Selektanten der Pilze der Gattung Cunninghamella zum Einsatz im erfindungsgemäßen Verfahren, solange sie die Verbindung 2 in eine Verbindung der Formel III und/oder II umsetzen. Ferner können Pilze der Gattung Cunninghamella zur Phosphorylierung der Verbindung 1 eingesetzt werden.

Die Nährlösung enthält Kohlenstoffquellen wie Saccharose, Maisstärke, Dextrose oder Molasse und Stickstoffquellen wie Sojabohnenmehl, Erdnußmehl, Malzextrakt oder Ammoniumacetat.

Das Nährmedium enthält auch anorganische Salze wie Natriumhydrogenphosphat, Natriumchlorid, Calciumchlorid, Calciumsulfat, Calciumcarbonat, Magnesiumsulfat oder Kaliumhydrogenphosphat. Ferner kann dem Nährmedium auch Fett wie Ölsäuremethylester oder Sojaöl zugesetzt werden. Daneben werden auch Spurenelemente wie Eisen-, Mangan-, Kupfer-, Zink-, Kobalt- oder andere Metallsalze zugegeben.

Die Kultivierung der Pilze erfolgt bei Temperaturen von 20°C bis 35°C, bevorzugt bei 28°C und bei pH-Werten von 5 bis 9, vorzugsweise bei pH 8. Die Kultivierung erfolgt aerob zunächst im Schüttelkolben und danach im Fermenter unter Rühren und Belüftung mit Luft oder reinem Sauerstoff. Die Kultivierung der Mikroorganismen in den Fermentern erfolgt über einen Zeitraum von 48 bis 240 Stunden, bevorzugt von 70 bis 110 Stunden.

Die Zugabe der Verbindung 2 erfolgt direkt zu Beginn der Kultivierung kann jedoch auch später erfolgen, bevorzugt nach etwa 48 Stunden. Die Zugabe der Verbindung 2 erfolgt als Festsubstanz, als Suspension oder in Lösung.

Geeignete Lösungsmittel sind Ethanol, Dimethylformamid (DMF), aber auch Dimethylsulfoxid (DMSO), Dimethylacetamid (DMA), Dimethoxyethan (DME), Tetrahydrofuran (THF) sowie Dibutyl-, Diisopropyl-, diethylformamid, 1-Methyl-, 1-Ethyl-, 1-Cyclohexylpyrrolidon, 4-Formylmorpholin, 1-Formylpiperidin, 1-Formylpyrrolidin, Tetramethyl-, Tetraethyl-, Tetrabutylharnstoff, Tripiperidino-, Tripyrrolidinophosphinoxid, Sulfolan oder N-Methylcaprolactam oder Mischungen der genannten Lösungsmittel.

Es können auch Lösungsvermittler wie ®Tween 80 oder Natriumdodecylsulfat zugefügt werden.

Die Isolierung der Verbindung der Formel III erfolgt direkt aus der Nährlösung oder nach Abtrennung der Zellen beispielsweise durch Zentrifugation oder Filtration. Die Isolierung der Verbindung der Formel III kann durch Extraktion mit Lösungsmitteln oder durch Adsorption an hydrophobe Harze wie XAD 16, HP 20 oder Ionenaustauscher erfolgen.

Für die Herstellung der Verbindung der Formel III, worin R¹ für -COOH und R² für -O-P(O)(OH)₂ steht wird bevorzugt Verbindung 1 als Substrat der Umsetzung eingesetzt.

Die Erfindung betrifft ferner neue Verbindungen der Formel II,worin 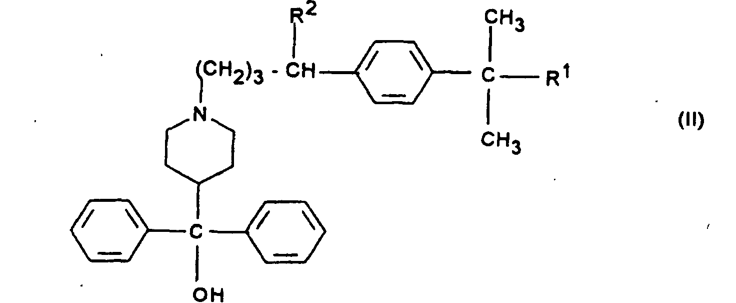
R¹ für -CH₂-O-P(O)(OH)₂ und R² für -OH (Verbindung 3) steht,
R¹ für -CH₃ und R² für -O-P(O)(OH)₂ (Verbindung 4) steht oder
R² für -O-P(O)(OH)₂ und R¹ für -COOH steht.

Die Erfindung betrifft auch Arzneimittel gekennzeichnet durch einen wirksamen Gehalt an mindestens einer Verbindung der Formel II und/oder eines physiologisch verträglichen Salzes der Verbindung der Formel II zusammen mit einem pharmazeutisch geeigneten und physiologisch verträglichen Trägerstoff.

Die Erfindung umfaßt auch pharmazeutisch verträgliche Salze der Verbindung der Formel II. Pharmazeutisch verträgliche Säureadditionssalze erfindungsgemäßer Verbindungen sind solche mit geeigneten anorganischen oder organischen Säuren. Geeignete anorganische Säuren sind zum Beispiel Salzsäure, Bromwasserstoffsäure, Schwefelsäure und Phosphorsäure. Zu den geeigneten organischen Säuren gehören Carbonsäuren wie Essigsäure, Propionsäure, Glycolsäure, Milchsäure, Brenztraubensäure, Malonsäure, Bernsteinsäure, Fumarsäure, Äpfelsäure, Weinsäure, Zitronensäure, Cyclamensäure, Ascorbinsäure, Hydroxymaleinsäure, Dihydroxymaleinsäure, Benzoesäure, Phenylessigsäure, 4-Aminobenzoesäure, 4-Hydroxybenzoesäure, Anthranilsäure, Zimtsäure, Salizylsäure, 4-Aminosalizylsäure, 2-Phenoxybenzoesäure, 2-Acetoxybenzoesäure und Mandelsäure, Sulfonsäuren wie Methansulfonsäure, Ethansulfonsäure und β-Hydroxyethansulfonsäure. Auch nicht-toxische Salze der Verbindungen obiger Formeln mit anorganischen oder organischen Basen fallen in den Rahmen der Erfindung. Hierzu gehören zum Beispiel die Salze mit Alkalimetallen wie Natrium, Kalium und Lithium, mit Erdalkalimetallen wie Calcium und Magnesium, Leichtmetallen der Gruppe IIIA wie Aluminium, mit organischen Aminen wie primären, sekundären und tertiären Aminen, zum Beispiel Cyclohexylamin, Ethylamin, Pyridin, Methylaminoethanol und Piperazin. Die Salze werden auf konventionelle Weise gebildet, zum Beispiel, indem man eine Verbindung der Formel II mit der entsprechenden Säure oder Base umsetzt.

Aufgrund der pharmakologischen Eigenschaften eignen sich die erfindungsgemäßen Verbindungen der Formel II als Antihistamine, Antiallergene und Bronchiendilatoren.

Die erfindungsgemäßen Verbindungen der Formel II sind geeignet zur Behandlung von allergischer Rhinitis, Asthma oder anderen allergischen Erkrankungen.

Die erfindungsgemäßen Verbindungen der Formel II können oral, parenteral, zum Beispiel subkutan, intravenös, intramuskulär, intraperitoneal, durch intranasale Einführung oder Applikation auf Schleimhäute, zum Beispiel der Nase, des Rachens oder des Bronchialtrakts, beispielsweise in Form eines Aerosol-Sprays, der kleine Partikel einer erfindungsgemäßen Verbindung in Nebel- oder Pulverform enthält, verabreicht werden.

Die Mengen der zu verabreichenden Verbindungen hängen vom Patienten und der Art der Verabreichung ab. Die zu verabreichenden Menge kann innerhalb eines breiten Bereichs schwanken, so daß sich Dosiseinheiten mit wirksamer Menge von etwa 0,01 bis 20 mg/kg Körpergewicht/Tag zur Erzielung des gewünschten Effekts ergeben. Beispielsweise kann die gewünschte Antihistamin-, Antiallergen- oder bronchienerweiternde Wirkung erzielt werden durch Aufnahme einer Dosiseinheit wie beispielsweise einer Tablette mit 5 bis 300 mg der erfindungsgemäßen Verbindung, bevorzugt 10 bis 200 mg, die 1- bis 4 mal täglich genommen wird.

Die Erfindung betrifft auch ein Verfahren zur Herstellung eines Arzneimittels, das dadurch gekennzeichnet, daß man mindestens eine Verbindung der Formel II mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Die erfindungsgemäßen Verbindungen der Formel II können auch als injizierbare Lösungen oder Suspensionen in einem physiologisch verträglichen Verdünnungsmittel mit einem pharmazeutischen Träger verabreicht werden, der eine sterile Flüssigkeit wie Wasser oder ein Öl ist, wobei oberflächenaktive und andere pharmazeutisch zulässige Hilfsmittel zugesetzt werden können. Beispiele geeigneter Öle sind solche aus Erdöl, tierischen, pflanzlichen oder synthetischen Ursprungs wie Erdnußöl, Sojabohnenöl oder Mineralöl. Im allgemeinen werden Wasser, Kochsalzlösung, wäßrige Dextroselösung oder ähnliche Zuckerlösungen und Glykole wie Propylenglykol oder Polyethylenglykol als flüssige Träger, insbesondere für injizierbare Lösungen, bevorzugt.

Zur Verwendung als Aerosole werden die erfindungsgemäßen Verbindungen in Lösung oder Suspension in einem Aerosol-Behälter zusammen mit geeigneten Treibmitteln, zum Beispiel Kohlenwasserstoff-Treibmitteln wie Propan, Butan oder Isobutan oder Kohlendioxid oder Stickstoff oder anderen ökologisch zulässigen Treibmitteln und üblichen Hilfsstoffen unter Druck gesetzt. Die Verbindungen können auch aus druckfreier Form verabreicht werden, zum Beispiel mit Vernebelungsgeräten.

Zur Verabreichung in Frage kommen Warmblütler, Vögel, Säugetiere, zum Beispiel Menschen, Katzen, Hunde, Pferde, Schafe, Rinder, Kühe, Schweine, Lämmer, Ratten, Mäuse und Meerschweinchen.

### Beispiel 1

### Stammhaltung

100 µl einer Sporensuspension der Cunninghamella Stämme Cunninghamella blakesleeana FH 001, Cunninghamella blakesleeana FH 002, Cunninghamella blakesleeana FH 003, Cunninghamella echinulata DSM 1905, Cunninghamella echinulata Variante elegans ATCC 8688a, Cunninghamella elegans DSM 1908 und Cunninghamella echinulata Variante echinulata ATCC 9244 werden auf einer Agarplatte (20 g/l Malzextrakt, 2 g/l Pepton und 20 g/l Agar pH 6,5) ausplattiert und 72 h bei 28 °C inkubiert. Anschließend kann die Platte über mindestens acht Wochen bei 4 °C gelagert werden.
Alternativ können die Stämme in dem gleichen Medium ohne Zusatz von Agar submers kultiviert werden. Nach einer Kultur über 72 h bei 28 °C wird die Kultur mit 20 % Glycerin versetzt und die Suspension bei etwa -120 °C über flüssigem Stickstoff gelagert.

### Beispiel 2

Hochdruckflüssigchromatographie (HPLC)-Analytik

| | | |
|---|---|---|
| Probenvorbereitung | Kulturbrühe 1:1 mit Methanol verdünnen und Suspension 30 Minuten rühren. Abtrennung der Klarphase durch Zentrifugation. | |
| Laufmittel A | 900 ml | H₂O |
| | 100 ml | Acetonitril |
| | 1 ml | Trifluoressigsäure (TFA) |
| Laufmittel B | 900 ml | Acetonitril |
| | 100 ml | H₂O |
| | 1 ml | TFA |
| Detektion | 215 nm | |
| Fluß | 1 ml/min | |
| Gradient | 0 min | 50% B |
| | 10 min | 80% B |
| | 15 min | 80% B |
| | 16 min | 50% B |
| | 21 min | 50% B |
| Säulentemperatur | 55 °C | |
| Säule | Merck RP 18 5 µm Licrospher 100 | |
| | Länge 25 cm Durchmesser 4 mm | |
| Retentionszeiten | Verbindung 1 | 5,4 Minuten (min) |
| | Verbindung 2 | 9 min |
| | Triol | 5,7 min |
| | Verbindung 3 | 3,5 min |
| | Verbindung 4 | 6,9 min |

### Beispiel 3

### Screening mit Cunninghamella Stämmen

100 ml Screeningmedium (5 g/l Sojapepton, 5 g/l Hefeextrakt, 5 g/l NaCl, 5 g/l K₂HPO₄, 3 g/l Agar und 20 g/l Glucose / getrennt autoklaviert, pH 5,0 in einem Erlenmeyerkolben mit 1000 ml Volumen) werden mit einem Agarstück eines der in Beispiel 1 genannten Stämme beimpft. Die Kultur erfolgt zunächst 48 h bei 28 °C und 180 Umdrehungen pro Minute (UpM) auf einer rotierenden Schüttelmaschine. Nach dieser Zeit erfolgt die Zugabe von 20 mg der Verbindung 2 gelöst in 100 µl DMF oder Ethanol. Nach 72 Stunden (h) oder wahlweise einem früheren Zeitpunkt wird ein Aliquot der Kultur entnommen und mit 50 % Methanol versetzt. Die erhaltene Suspension wird mit einem Ultra-Turrax 30 Sekunden suspendiert und die Suspension anschließend bei 10 000 g zentrifugiert. Der erhaltene klare Überstand wird dann mittels HPLC (siehe Beispiel 2) analysiert.
In der Kulturbrühe von allen Stämmen konnte nach 72 h Inkubationszeit die Verbindung 1 in einer Konzentration von bis zu 5 mg/l nachgewiesen werden.

### Beispiel 4

### Produktion der Verbindung 1 im Kolbenmaßstab mit verschiedenen Cunninghamella Stämmen

100 ml Vorkulturmedium (5 g/l Sojapepton, 5 g/l Hefeextrakt, 5 g/l NaCl, 5 g/l K₂HPO₄, 3 g/l Agar und 20 g/l Glucose / getrennt autoklaviert, pH 5,0 in einem Erlenmeyerkolben mit 1000 ml Volumen) werden mit einem Agarstück, auf dem jeweils einer der in Beispiel 1 genannten Stämme gewachsen ist, beimpft. Die Kultur erfolgt 48 h bei 28 °C und 180 UpM auf einer rotierenden Schüttelmaschine. 100 ml Produktionsmedium (5 g/l Sojapepton, 10 g/l Cornsteep flüssig, 20 g/l Glucose, 5 g/l NaCl, 1 g/l K₂HPO₄, 3 g/l Agar, 25,1 g/l 2-([Hydroxy-1,1-bis-(hydroxymethyl)ethyl]-amino)ethansulfonsäure (TES) (Natriumsalz), 100 µl Desmophen, pH 8,0 in einem Erlenmeyerkolben mit 500 ml Volumen) werden mit 10 ml der oben beschriebenen Vorkultur beimpft. Die Kultur erfolgt zunächst 48 h bei 28 °C und 180 UpM auf einer rotierenden Schüttelmaschine.
Nach dieser Zeit erfolgt die Zugabe von 20 mg Verbindung 1 gelöst in 100 µl DMF. Nach 72 h wird ein Aliquot der Kultur entnommen und mit 50 % Methanol versetzt. Die erhaltene Suspension wird mit einem Ultra-Turrax 30 Sekunden suspendiert und die Suspension anschließend bei 10 000 g zentrifugiert. Der erhaltene klare Überstand wird dann mittels HPLC (Beispiel 2) analysiert.
Die Analyse ergibt die in der Tabelle 1 zusammengestellten Ausbeuten; es werden folgende Verbindungen gefunden:
Verbindung 1 ist die Verbindung der Formel I;
Verbindung 2 ist die Verbindung der Formel II, worin R¹ für -CH₃ und R² für -OH steht;
Verbindung 3 ist die Verbindung der Formel II, worin R¹ für -CH₂-O-P(O)(OH)₂ und R² für -OH steht;
Verbindung 4 ist die Verbindung der Formel II, worin R¹ für -CH₃ und R² für -O-P(O)(OH)₂ steht und
Triol ist die Verbindung der Formel II, worin R¹ für -OH und R² für -OH stehen.

**Tabelle 1**

| Stamm | Triol [mg/l] | Verbindung 1 [mg/l] | Verbindung 3 [mg/l] | Verbindung 4 [mg/l] |
|---|---|---|---|---|
| C. blak. FH 001 | 4,9 | < 1,0 | 11,9 | 60,4 |
| C. blak. FH 002 | 26,0 | 50,7 | 42,5 | 3,0 |
| C. blak. FH 003 | 2,1 | < 1,0 | 1,6 | 18,4 |
| C. echinu. ATCC 8688a | 4,7 | 43,2 | 27,3 | 2,2 |
| C. echinu. DSM 1905 | 13,8 | 3,3 | 40,4 | 23,1 |
| C. el. DSM 1908 | 17,3 | 1,1 | 15,2 | 37,0 |
| C. ech. ATCC 9244 | 18,5 | 5,1 | 20,7 | 7,4 |

### Beispiel 5

### Verbesserte Herstellung der Verbindung 1 im Kolbenmaßstab mit Cunninghamella echinulata Variante elegans ATCC 8688a

100 ml Vorkulturmedium (5 g/l Sojapepton, 5 g/l Hefeextrakt, 5 g/l NaCl, 5 g/l K₂HPO₄, 3 g/l Agar und 20 g/l Glucose (getrennt autoklaviert), pH 5,0 in einem Erlenmeyerkolben mit 1000 ml Volumen) werden mit einem Agarstück, auf dem jeweils einer der in Beispiel 1 genannten Stämme gewachsen ist, beimpft. Die Kultur erfolgt 48 h bei 28 °C und 180 UpM auf einer rotierenden Schüttelmaschine. 100 ml Produktionsmedium (5 g/l Sojapepton, 5 g/l Caseinpepton, 20 g/l lösliche Stärke, 5 g/l NaCl, 1 g/l K₂HPO₄, 1 g/l NH₄(SO₄)₂, 3 g/l Agar, 25,1 g/l TES (Natriumsalz), 100 µl Desmophen, pH 8,0 in einem Erlenmeyerkolben mit 500 ml Volumen) werden mit 10 ml der oben beschriebenen Vorkultur beimpft. Die Kultur erfolgt zunächst 24 h bei 28°C und 180 UpM auf einer rotierenden Schüttelmaschine.
Nach dieser Zeit erfolgt die Zugabe von 20 mg Verbindung 2 gelöst in 800 µl Ethanol. Nach 72 h wird ein Aliquot der Kultur entnommen und mit 50 % Methanol versetzt. Die erhaltene Suspension wird mit einem Ultra-Turrax 30 Sekunden suspendiert und die Suspension anschließend bei 10 000 g zentrifugiert. Der erhaltene klare Überstand wird dann mittels HPLC (Beispiel 2) analysiert.
Die Analyse ergab eine Ausbeute von 145 mg/l Verbindung 1, 6,5 mg/l Triol (2), 22 mg/l Verbindung 3 und 5 mg/l Verbindung 4.

### Beispiel 6

### Produktion von Verbindungen der Formel II im 10 I-Fermenter

100 ml Vorkulturmedium (5 g/l Sojapepton, 5 g/l Hefeextrakt, 5 g/l NaCl, 5 g/l K₂HPO₄, 3 g/l Agar und 20 g/l Glucose / getrennt autoklaviert, pH 5,0 in einem Erlenmeyerkolben mit 1000 ml Volumen) werden mit einem Agarstück des Stammes Cunninghamella echinulata Var. elegans ATCC 8688a beimpft. Die Kultur erfolgt 48 h bei 28 °C und 180 UpM auf einer rotierenden Schüttelmaschine.
10 l Produktionsmedium (5 g/l Sojapepton, 5 g/l Hefeextrakt, 20 g/l Glucose (getrennt autoklaviert), 5 g/l NaCl, 1 g/l K₂HPO₄, 100 µl Desmophen, pH 5,0) werden mit 100 ml der oben beschriebenen Vorkultur beimpft. Anschließend wird die Verbindung 2 dazugegeben, so daß eine Endkonzentration im Fermenter von 200 mg/ l Produktionsmedium erreicht wird. Die Fermentation erfolgt bei 28 °C, 700 UpM und 0,5 wm über 96 Stunden.
Nach HPLC-Analyse enthält die Kulturbrühe etwa 10 mg/l Verbindung 1, 10 mg/l Triol (2), 100 mg/l Verbindung 3 und 100 mg/l Verbindung 4.

### Beispiel 7

### Isolierung der Verbindungen der Formel II

Die nach Beispiel 6 erhaltene Kulturbrühe (8800 ml) wurde mittels Becherzentrifuge (5000 g /10 Minuten) in Überstand (8000 ml) und Biomasse (800 g) getrennt und beide Phasen wurden separat aufgearbeitet.
Dem Überstand wurden 160 g makroporöses Acrylamid-Polystyrol Adsorberharz wie XAD 7 zugesetzt. Die Adsorption erfolgte unter leichtem Rühren bei pH 2,5 und Raumtemperatur über 2 Stunden. Das Adsorberharz wurde abfiltriert und mit 4 mal 160 ml Methanol eluiert. Die Eluate wurden vereinigt und unter verminderten Druck zur Trockene eingeengt (etwa 50 g öliger Feststoff nach Lyophilisation).
Die Feinreinigung erfolgte mittels präparativer HPLC nach folgendem Verfahren:

| | | |
|---|---|---|
| Laufmittel A : | 800 ml | H₂O |
| | 200 ml | Acetonitril |
| | 1 ml | TFA |
| Laufmittel B : | 1000 ml | Acetonitril |
| | 0,9 ml | TFA |
| Detektion : | 215 nm | |
| Fluß : | 50 ml/min | |
| Gradient : | 0 min | 0% B |
| | 60 min | 0% B |
| | 133 min | 43% B |
| | 134 min | 100% B |
| | 157 min | 100% B |
| Säulentemperatur: | Raumtemperatur | |
| Säule : | Kromasil, C18, 7 µm | |
| | Länge 350 mm Durchmesser 50 mm | |
| Probe | 5 g Rohprodukt, gelöst in 350 ml Methanol / Wasser (1:1) | |

Aus zwei Läufen werden nach Vereinigung der entsprechenden Fraktionen, Einengen unter verminderten Druck und Lyophilisation des wäßrigen Rückstandes 20 mg Verbindung 1 und 160 mg Verbindung 3 erhalten.

Die Biomasse (800 g) wird mit n-Propanol 30%ig und 100%ig (insgesamt 1500 ml) extrahiert und der Feststoff mittels Filtration über Filterschichten (K300) abgetrennt. Die Filtrate werden unter verminderten Druck eingeengt und 26,2 g öliger Feststoff erhalten.
Die Feinreinigung erfolgt mittels präparativer HPLC nach folgendem Verfahren:

| | | |
|---|---|---|
| Laufmittel A : | 800 ml | H₂O |
| | 200 ml | Acetonitril |
| | 1 ml | TFA |
| Laufmittel B: | 1000 ml | Acetonitril |
| | 0,9 ml | TFA |
| Detektion: | 215 nm | |
| Fluß: | 50 ml/min | |
| Gradient: | 0 min | 0% B |
| | 34 min | 0% B |
| | 121 min | 51% B |
| | 122 min | 100% B |
| | 148 min | 100% B |
| Säulentemperatur: | Raumtemperatur | |
| Säule: | Kromasil, C18, 7 µm | |
| | Länge 350 mm Durchmesser 50 mm | |
| Probe | 5 g Rohprodukt, gelöst in 400 ml Methanol / Wasser (1:1) | |

Aus zwei Läufen werden nach Vereinigung der entsprechenden Fraktionen, Einengen unter verminderten Druck und Lyophilisation des wäßrigen Rückstandes 4 mg Triol (2), 20 mg Verbindung 4 und 80 mg Verbindung 1 erhalten.

### Beispiel 8

### Charakterisierung der Verbindungen der Formel II

Neben der Charakterisierung mittels UV/VIS Spektroskopie werden die Komponenten durch ¹H-NMR und ESI-MS oder FAB-MS charakterisiert.
MS-Daten:

| | |
|---|---|
| Triol (2) | ESI-MS: m/e = 488.4 [M+H]⁺ |
| Verbindung 1 | ESI-MS: m/e = 502.3 [M+H]⁺ |
| Verbindung 3 | ESI-MS: m/e = 568.3 [M+H]⁺ HR-FAB-MS: m/e = 568.2827 [M+H]⁺ |
| Verbindung 4 | ESI-MS: m/e = 552.3 [M+H]⁺ |

¹H-NMR Daten werden in Tabelle 2 dargestellt:
¹H-chemische Verschiebungen in DMSO bei 300 °K.

**Tabelle 2:**

| | Verbindung 1 | Verbindung 3 | Triol 2 | Verbindung 4 |
|---|---|---|---|---|
| 1-CH₃ | 1.45 | 1.25 | 1.20 | 1.25 |
| 1-CH₂ | - | 3.83 | 3.39 | - |
| 3 | 7.30 | 7.34 | 7.30 | 7.32 |
| 4 | 7.28 | 7.26 | 7.23 | 7.27 |
| 6 | 4.52 | 4.52 | 4.51 | 5.11 |
| 7 | 1.66/1.59 | 1.68/1.59 | 1.68/1.58 | 1.71 |
| 8 | 1.59 | 1.58 | 1.58 | 1.73 |
| 9 | 2.98 | 2.98 | 2.97 | 2.97 |
| 10 | 3.43/2.89 | 3.43/2.89 | 3.41/2.88 | 3.31/2.83 |
| 11 | 1.66/1.46 | 1.66/1.45 | 1.66/1.46 | 1.74/1.37 |
| 12 | 2.83 | 2.83 | 2.82 | 2.81 |
| 15 | 7.49 | 7.49 | 7.49 | 7.50 |
| 16 | 7.28 | 7.29 | 7.30 | 7.28 |
| 17 | 7.16 | 7.16 | 7.16 | 7.15 |

### Beispiel 9

### Wirkung auf die Bronchialweite des narkotisierten Meerschweinchens

Die Untersuchungen erfolgten gemäß der Methode von Konzett-Rössler (Arch. exp. Path. U. Pharmak. 195 (71-74) 1940), an männlichen Albino-Meerschweinchen mit einem Körpergewicht von 312-415 g. Die Versuchspräparate wurden in einer Dosis von 10 mg/kg in einer Suspension mit dem Verabreichungsvolumen von 2 ml/kg Körpergewicht appliziert. Nach zwei Vorwerten mit Histamin 20 µg/kg intravenös (i.v.) wurde die Substanz einmalig mittels einer Zwölffingerdarmsonde eingegeben und die Asthmatestung 15, 30, 60, 90, 120, 150, 180, 210 und 240 min nach Präparatgabe wiederholt.
Die Änderungen der Bronchialweite im Vergleich zur Kontrollmessung wurde prozentual angegeben. Pro Dosis wurden 6 Tiere eingesetzt. Die Statistik erfolgte über den t-Test.

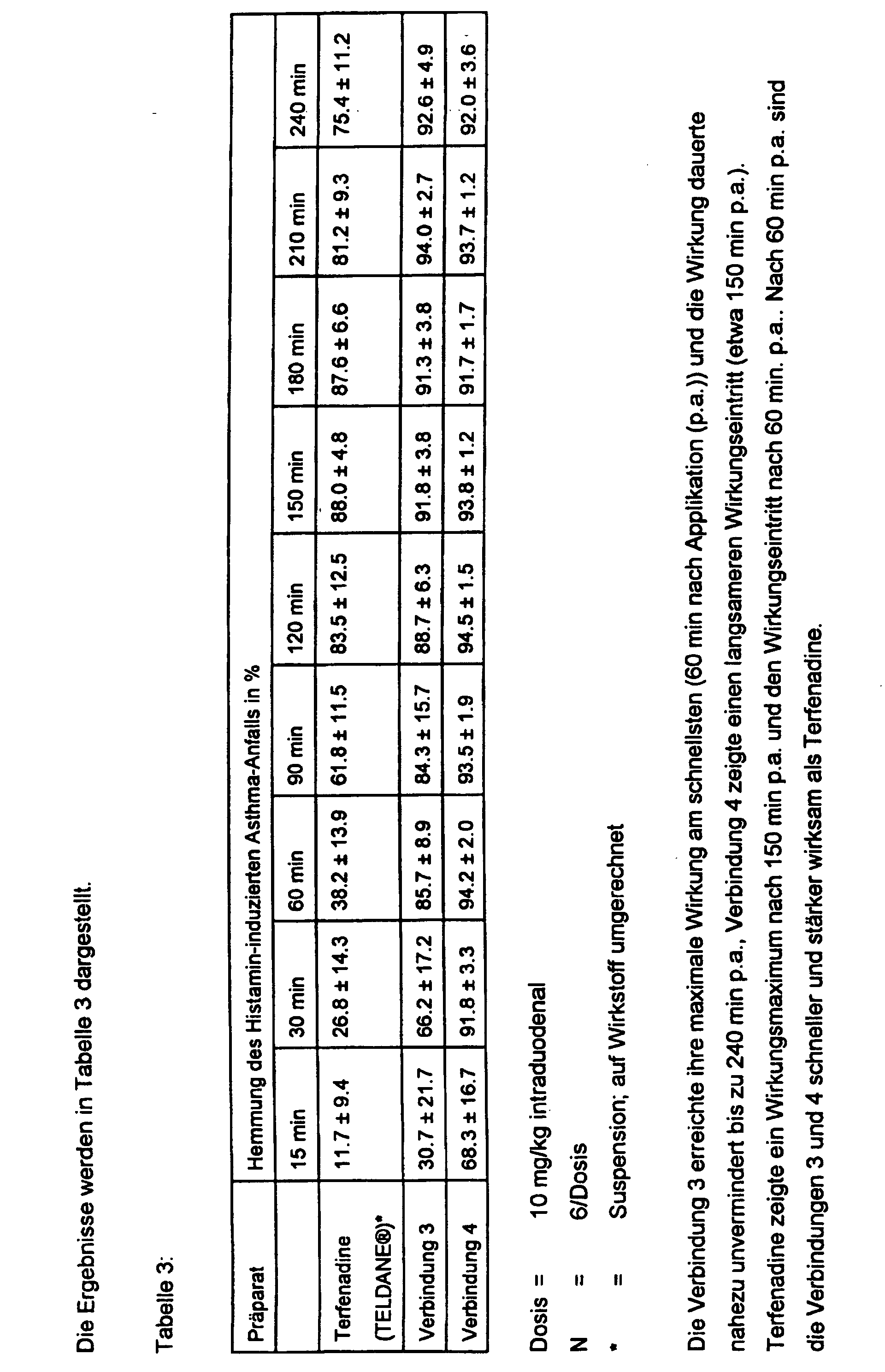

## Patentansprüche

1. Verbindung der Formel II, 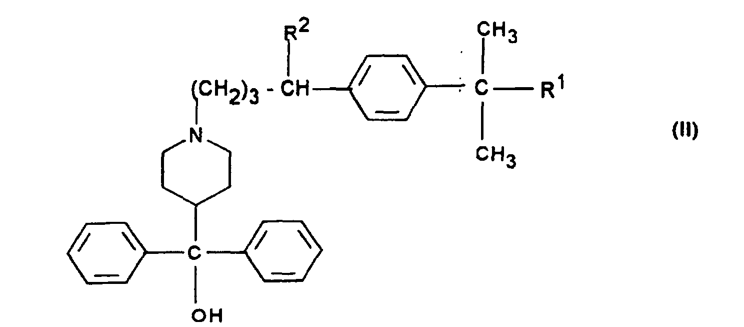 worin
R¹ für -CH₂-O-P(O)(OH)₂ und R² für -OH steht,
R¹ für -CH₃ und R² für -O-P(O)(OH)₂ steht oder
R² für -O-P(O)(OH)₂ und R¹ für -COOH steht.

2. Verfahren zur Herstellung der Verbindung der Formel III, 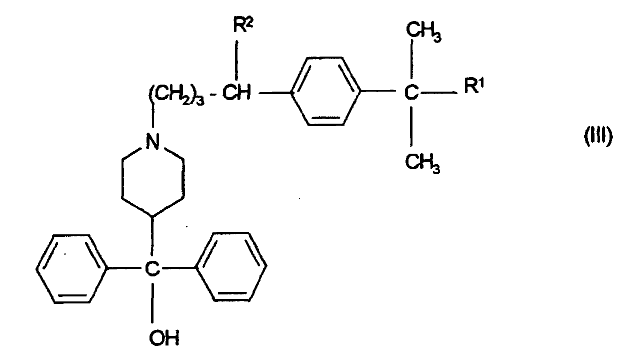 worin
R¹ für -CH₂-O-P(O)(OH)₂ und R² für -OH steht ,
R¹ für -CH₃ und R² für -O-P(O)(OH)₂ steht oder
R² für -O-P(O)(OH)₂ und R¹ für -COOH steht,
**dadurch gekennzeichnet, daß** α-(p-tertiär-Butylphenyl)-4-(α-hydroxy-α-phenylbenzyl)-1-piperidin-butanol oder gegebenenfalls 4-(4-(4-Hydroxydiphenyl)-1-piperidinyl)-1-hydroxybutyl)-α,α-dimethylessigsäure mit einem Pilz der Gattung Cunninghamella oder Absidia inkubiert wird.

3. Arzneimittel, **gekennzeichnet durch** einen wirksamen Gehalt an mindestens einer Verbindung der Formel II gemäß Anspruch 1 zusammen mit einem pharmazeutisch geeigneten und physiologisch verträglichen Trägerstoff.

4. Verwendung von mindestens einer Verbindung der Formel II gemäß Anspruch 1 zur Herstellung von Arzneimitteln zur Behandlung von allergischen Erkrankungen, allergischer Rhinitis oder Asthma.

5. Verfahren zur Herstellung eines Arzneimittels gemäß Anspruch 3, **dadurch gekennzeichnet, daß** man mindestens eine Verbindung gemäß Anspruch 1 und einen pharmazeutisch geeigneten und physiologisch verträglichen Trägerstoff in eine geeignete Darreichungsform bringt.

6. Verfahren gemäß Anspruch 2, **dadurch gekennzeichnet, daß** ein Pilz aus der Gruppe Cunninghamella blakesleeana, Cunninghamella elegans und Cunninghamella echinulata, insbesondere Cunninghamella echinulata Variante elegans ATCC 8688a, Cunninghamella echinulata DSM 1905, Cunninghamella elegans DSM 1908 und Cunninghamella echinulata ATCC 9244, eingesetzt wird.

7. Verfahren nach einem oder mehreren der Ansprüche 2 oder 6, **dadurch gekennzeichnet, daß** α-(p-tert.-Butylphenyl)-4-(α-hydroxy-α-phenylbenzyl)-1-piperidin-butanol zu Beginn der Kultivierung des Pilzes der Gattung Cunninghamella oder 24 Stunden nach Beginn zugegeben wird.

## Claims

1. A compound of the formula II 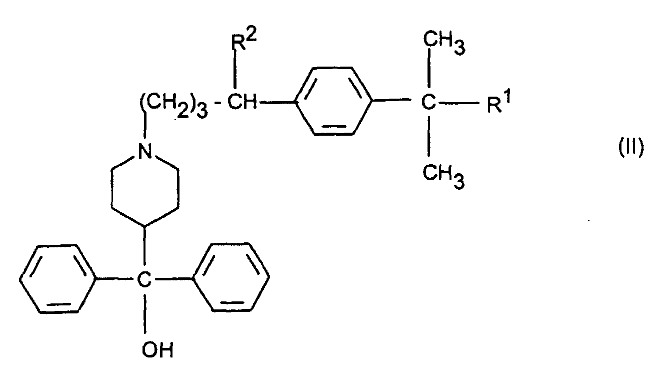 in which
R¹ is -CH₂-O-P(O)(OH)₂ and R² is -OH,
R¹ is -CH₃ and R² is -O-P(O)(OH)₂ or
R² is -O-P(O)(OH)₂ and R¹ is -COOH.

2. A process for the preparation of the compound of the formula III 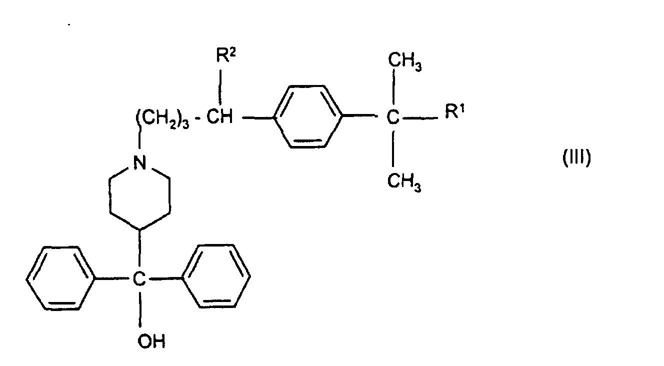 in which
R¹ is -CH₂-O-P(O)(OH)₂ and R² is -OH,
R¹ is -CH₃ and R² is -O-P(O)(OH)₂ or
R² is -O-P(O)(OH)₂ and R¹ is -COOH,
which comprises incubating α-(p-tertiary-butylphenyl)-4-(α-hydroxy-α-phenylbenzyl)-1-piperidinylbutanol or, if appropriate, 4-(4-(4-hydroxydiphenyl)-1-piperidinyl)-1-hydroxybutyl)-α,α-dimethylacetic acid with a fungus of the genus Cunninghamella or Absidia.

3. A pharmaceutical comprising an effective amount of at least one compound of the formula II as claimed in claim 1 together with a pharmaceutically suitable and physiologically tolerable excipient.

4. The use of at least one compound of the formula II as claimed in claim 1 for the production of pharmaceuticals for the treatment of allergic disorders, allergic rhinitis or asthma.

5. A process for the production of a pharmaceutical as claimed in claim 3, which comprises bringing at least one compound as claimed in claim 1 and a pharmaceutically suitable and physiologically tolerable excipient into a suitable administration form.

6. A process as claimed in claim 2, wherein a fungus from the group consisting of Cunninghamella blakesleeana, Cunninghamella elegans and Cunninghamella echinulata, in particular Cunninghamella echinulata variant elegans ATCC 8688a, Cunninghamella echinulata DSM 1905, Cunninghamella elegans DSM 1908 and Cunninghamella echinulata ATCC 9244, is employed.

7. A process as claimed in one or both of claims 2 and 6, wherein α-(p-tert-butylphenyl)-4-(α-hydroxy-α-phenylbenzyl)-1-piperidinylbutanol is added at the start of the culturing of the fungus of the genus Cunninghamella or 24 hours after the start.

## Revendications

1. Composé de formule II 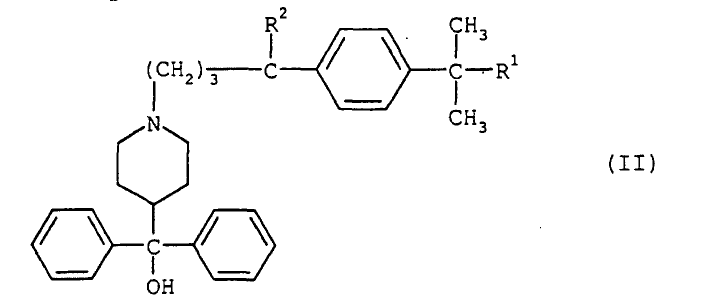 dans laquelle
R¹ est -CH₂-O-P(O)(OH)₂ et R² est -OH,
R¹ est -CH₃ et R² est -O-P(O)(OH)₂, ou bien
R² est -O-P(O)(OH)₂ et R¹ est -COOH.

2. Procédé de préparation du composé de formule III 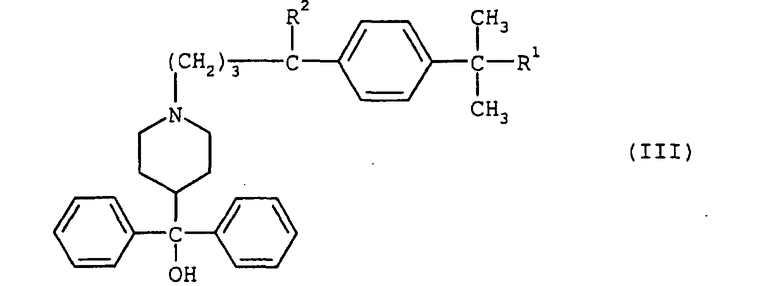 dans laquelle
R¹ est -CH₂-O-P(O)(OH)₂ et R² est -OH,
R¹ est -CH₃ et R² est -O-P(O)(OH)₂, ou bien
R² est -O-P(O)(OH)₂ et R¹ est -COOH.
**caractérisé en ce qu'**on incube avec un champignon du genre Cunninghamella ou Absidia de l'a-(p-tert-butylphényl)-4-(α-hydroxy-α-phénylbenzyl)-1-pipéridine-butanol ou éventuellement de l'acide 4-(4-(4-hydroxydiphényl)-1-pipéridinyl)-1-hydroxybutyl)-α,α-diméthylacétique.

3. Médicament, **caractérisé en ce qu'**il contient une quantité active d'au moins un composé de formule II selon la revendication 1, en même temps qu'un excipient acceptable d'un point de vue pharmaceutique et compatible d'un point de vue physiologique.

4. Utilisation d'au moins un composé de formule II selon la revendication 1 pour préparer des médicaments destinés au traitement de maladies allergiques, de la rhinite allergique ou de l'asthme.

5. Procédé de préparation d'un médicament selon la revendication 3, **caractérisé en ce qu'**on met sous une forme posologique appropriée un composé selon la revendication 1 et un excipient acceptable d'un point de vue pharmaceutique et compatible d'un point de vue physiologique.

6. Procédé selon la revendication 2, **caractérisé en ce qu'**on utilise un champignon choisi dans l'ensemble comprenant Cunninghamella blakesleeana, Cunninghamella elegans et Cunninghamella echinulata, en particulier Cunninghamella echinulata variante elegans ATCC 8688a, Cunninghamella echinulata DSM 1905, Cunninghamella elegans DSM 1908 et Cunninghamella echinulata ATCC 9244.

7. Procédé selon l'une ou plusieurs des revendications 2 ou 6, **caractérisé en ce qu'**on ajoute l'α-(p-tert-butylphényl)-4-(α-hydroxy-α-phénylbenzyl)-1-pipéridine-butanol au début de la culture du champignon du genre Cunninghamella, ou 24 heures après le début.
